# EUROPEAN PATENT SPECIFICATION

(11) **EP 4 347 899 B1**
(45) Date of publication and mention of the grant of the patent: **18.11.2026**
(21) Application number: 22726530.3
(22) Date of filing: 19.05.2022
(51) Int. Cl.: C21B 13/00, C21B 13/02, C07C 1/12, C25B 1/04, C25B 15/08

(54) **A METHOD FOR MANUFACTURING DIRECT REDUCED IRON**
VERFAHREN ZUR HERSTELLUNG VON DIREKT REDUZIERTEM EISEN
PROCÉDÉ DE FABRICATION D'ÉPONGE DE FER

(30) Priority: 26.05.2021 WO PCT/IB2021/054583
(43) Date of publication of application: 10.04.2024
(73) Proprietor: ArcelorMittal, 1160 Luxembourg (LU)
(72) Inventor: SALAME, Sarah, 57283 Maizieres Les Metz (FR); CARRIER, Odile, 57000 Metz (FR); BARROS LORENZO, José, 57283 Maizieres Les Metz (FR); REYES RODRIGUEZ, Jon, 33400 Aviles Asturias (ES); ANDRADE, Marcelo, East Chicago, IN 46312 (US); BOULANOV, Dmitri, East Chicago, IN 46312 (US); LU, Dennis, East Chicago, IN 46312-2939 (US); TSVIK, George, Valparaiso, IN 46385 (US)
(74) Representative: Lavoix
(86) International application number: PCT/IB2022/054664
(87) International publication number: WO 2022/248987

(56) References cited:
- US-A1- 2016 083 811
- US-A1- 2017 002 433
- US-A1- 2018 363 075
- URIBE-SOTO WILMAR ET AL: "A review of thermochemical processes and technologies to use steelworks off-gases", RENEWABLE AND SUSTAINABLE ENERGY REVIEWS, ELSEVIERS SCIENCE, NEW YORK, NY, US, vol. 74, 9 March 2017 (2017-03-09), pages 809 - 823, XP029986176, ISSN: 1364-0321, DOI: 10.1016/J.RSER.2017.03.008

## Description

The invention is related to a method for manufacturing Direct Reduced Iron (DRI) and to a DRI manufacturing equipment
Steel can be currently produced through two main manufacturing routes. Nowadays, most commonly used production route consists in producing pig iron in a blast furnace, by use of a reducing agent, mainly coke, to reduce iron oxides. In this method, approx. 450 to 600 kg of coke, is consumed per metric ton of pig iron; this method, both in the production of coke from coal in a coking plant and in the production of the pig iron, releases significant quantities of CO2.

The second main route involves so-called "direct reduction methods". Among them are methods according to the brands MIDREX, FINMET, ENERGIRON/HYL, COREX, FINEX etc., in which sponge iron is produced in the form of HDRI (Hot Direct Reduced Iron), CDRI (cold direct reduced iron), or HBI (hot briquetted iron) from the direct reduction of iron oxide carriers. Sponge iron in the form of HDRI, CDRI, and HBI usually undergo further processing in electric arc furnaces. Documents "A review of thermochemical processes and technologies to use steelworks off-gases" from URIBE-SOTO WILMAR ET AL (DOI: 10.1016/J.RSER.2017.03.008) and US 2018363075 further disclose alternative methods for manufacturing direct reduced iron.

There are three zones in each direct reduction shaft with cold DRI discharge: Reduction zone at top, transition zone at the middle, cooling zone at the cone shape bottom. In hot discharge DRI, this bottom part is used mainly for product homogenization before discharge.

Reduction of the iron oxides occurs in the upper section of the furnace, at temperatures up to 950°C. Iron oxide ores and pellets containing around 30% by weight of Oxygen are charged to the top of a direct reduction shaft and are allowed to descend, by gravity, through a reducing gas. This reducing gas is entering the furnace from the bottom of reduction zone and flows counter-current from the charged oxidised iron. Oxygen contained in ores and pellets is removed in stepwise reduction of iron oxides in counter-current reaction between gases and oxide. Oxidant content of gas is increasing while gas is moving to the top of the furnace.

The reducing gas generally comprises hydrogen and carbon monoxide (syngas) and is obtained by the catalytic reforming of natural gas. For example, in the so-called MIDREX method, first methane is transformed into a reformer according to the following reaction to produce the syngas or reduction gas:

CH4 + CO2 -> 2CO + 2H2

and the iron oxide reacts with the reduction gas, for example according to the following reactions:

3Fe203 + CO/H2 -> 2Fe3O4+CO2/H2O

Fe3O4 + CO/H2 -> 3 FeO + CO2/H2O

FeO + CO/H2 -> Fe + CO2/H2O

At the end of the reduction zone the ore is metallized.

A transition section is found below the reduction section; this section is of sufficient length to separate the reduction section from the cooling section, allowing an independent control of both sections. In this section carburization of the metallized product happens. Carburization is the process of increasing the carbon content of the metallized product inside the reduction furnace through following reactions:

3Fe + CH4 → Fe3C + 2H2 (Endothermic)

3Fe + 2CO → Fe3C + CO2 (Exothermic)

3Fe + CO + H2 → Fe3C + H2O (Exothermic)

Injection of natural gas in the transition zone is using sensible heat of the metallized product in the transition zone to promote hydrocarbon cracking and carbon deposition. Due to relatively low concentration of oxidants, transition zone natural gas is more likely to crack to H2 and Carbon than reforming to H2 and CO. Natural gas cracking provides carbon for DRI carburization and, at the same time adds reductant (H2) to the gas that increases the gas reducing potential.

Gas injection is also performed into cooling zone, it usually consists in recirculating cooling gas plus added natural gas. Natural gas (NG) addition to cooling gas allows operator to keep the recirculating cooling gas circuit with a high content in methane, otherwise, the predominant component in the cooling gas would be Nitrogen. The heat capacity of natural gas is much more than N2: cooling gas recirculating flow is 500-600 Nm3/t with NG, and 800 Nm3/t without NG. Although there will not be too much carbon deposition in cooling zone, but the up flow of cooling gas to higher levels of the furnace will provide more hydrocarbon for cracking.

In view of the considerable increase in the concentration of CO2 in the atmosphere since the beginning of the last century and the subsequent greenhouse effect, it is essential to reduce emissions of CO2 where it is produced in a large quantity, and therefore in particular during DRI manufacturing.

One solution which is currently developed is the progressive increase of the hydrogen content into the reducing gas, in view of reaching a pure hydrogen reducing gas. Following reduction reaction will then occur:

Fe2O3 + 3 H2 = 2 Fe + 3 H2O

thus releasing harmless H2O instead of the greenhouse gas CO2.

This however implies that the content of carbon into the reducing gas will be reduced and at some point, no more carbon will be injected into the shaft. As explained above this has an impact on the DRI product which will have a smaller and smaller carbon content.

Content of carbon in the DRI product is a key parameter at it plays an important role into the subsequent steps, such as slag foaming at the electric Arc furnace, but it also helps to improve the transportability of the DRI product.

Solutions are already known to increase the carbon content of the product, they mainly consist in injecting hydrocarbons into the shaft, usually CH4, or coke oven gas. But those gases will contribute to increase the carbon footprint of the DRI process which is not in line with the switch to pure H2 reduction.

There is a need for a method allowing to increase carbon content in the DRI product. There is also a need for a method allowing to further reduce the carbon footprint of the process.

This problem is solved by a method according to the invention, as disclosed in appended claims 1-13.

The method of the invention may also comprise the following optional characteristics considered separately or according to all possible technical combinations:
- the carbon-bearing liquid is injected at least into the transition zone,
- the carbon-bearing liquid is injected at least into the cooling zone,
- the carbon-bearing liquid is injected in the transition zone and in the cooling zone,
- the carbon-bearing liquid is a biofuel,
- the carbon-bearing liquid is liquid alcohol,
- the carbon-bearing liquid is liquid hydrocarbon,
- the carbon-bearing liquid is liquid ethanol
- the reducing gas comprises more than 50% in volume of hydrogen,
- the reducing gas comprises more than 99% in volume of hydrogen,
- the hydrogen of the reducing gas is at least partly produced by electrolysis,
- the electrolysis is powered by renewable energy,
- the CO2-rich gas is subjected to a hydrocarbon production step.

Other characteristics and advantages of the invention will emerge clearly from the description of it that is given below by way of an indication and which is in no way restrictive, with reference to the appended figures in which:
- Figure 1 illustrates a layout of a direct reduction plant allowing to perform a method according to the invention
- Figure 2A and 2B are curves simulating the increase of the carbon content into the DRI product when injecting liquid Ethanol or Methanol Elements in the figures are illustration and may not have been drawn to scale.

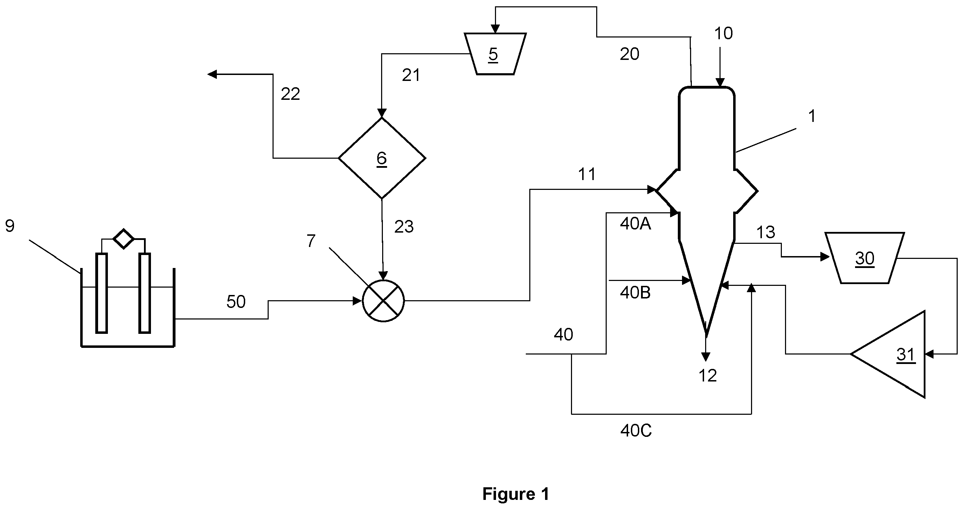

Figure 1 illustrates a layout of a direct reduction plant allowing to perform a method according to the invention.

The DRI manufacturing equipment includes a DRI shaft 1 comprising from top to bottom an inlet for iron ore 10 that travels through the shaft 1 by gravity, a reduction section located in the upper part of the shaft, a transition section located in the midpart of the shaft, a cooling section located at the bottom and an outlet from which the direct reduced iron 12 is finally extracted.

In the method according to the invention, the direct reduction furnace (or shaft) 1 is charged at its top with oxidized iron 10. This oxidized iron 10 is reduced into the furnace 1 by a reducing gas 11 injected into the furnace and flowing counter-current from the oxidized iron. Reduced iron 12 exits the bottom of the furnace 1 for further processing, such as briquetting, before being used in subsequent steelmaking steps. Reducing gas, after having reduced iron, exits at the top of the furnace as a top reduction gas 20 (TRG).

A cooling gas 13 can be captured out of the cooling zone of the furnace, subjected to a cleaning step into a cleaning device 30, such as a scrubber, compressed in a compressor 31 and then sent back to the cooling zone of the shaft 1.

In the method according to the invention, a carbon-bearing liquid 40 is injected below the reduction zone of the shaft 1. It may be injected in the transition zone, as illustrated by stream 40A and/or in the cooling zone, as illustrated by streams 40B and 40C. It may be injected alone 40B or in combination 40C with the cooling gas 13.

By carbon-bearing liquid it is meant a liquid product comprising carbon. It may be an alcohol, such as methanol or ethanol, or a hydrocarbon, such as methane. It may be of fossil or non-fossil origin; in a preferred embodiment it is a biofuel. By biofuel it is meant a fuel that is produced through processes from biomass, rather than by the very slow geological processes involved in the formation of fossil fuels, such as oil. Biofuel can be produced from plants (i.e. energy crops), or from agricultural, commercial, domestic, and/or industrial wastes (if the waste has a biological origin). This biofuel may preferentially be produced by conversion of steelmaking gases.

Once injected into the shaft, the carbon-bearing liquid 40 is cracked by the heat released by hot DRI, this producing a reducing gas and carburizing the DRI product to increase its carbon content. Moreover, the vaporization enthalpy further contributes to the DRI cooling.

The injection of this liquid is made to increase the carbon content of the Direct Reduced Iron to a range from 0.5 to 3 wt. %, preferably from 1 to 2 wt.% which allows getting a Direct Reduced Iron that can be easily handled and that keeps a good combustion potential for its future use.

In a preferred embodiment, the reducing gas 11 comprises at least 50%v of hydrogen, and more preferentially more than 99%v of H2. An H2 stream 40 may be supplied to produce said reducing gas 11 by a dedicated H2 production plant 9, such as an electrolysis plant. It may be a water or steam electrolysis plant. It is preferably operated using CO₂ neutral electricity which includes notably electricity from renewable source which is defined as energy that is collected from renewable resources, which are naturally replenished on a human timescale, including sources like sunlight, wind, rain, tides, waves, and geothermal heat. In some embodiments, the use of electricity coming from nuclear sources can be used as it is not emitting CO₂ to be produced.

In another embodiment, H2 stream 40 may be mixed with part of the top reduction gas 20 to form the reducing gas 11. When operated with natural gas the top reduction gas 20 usually comprises from 15 to 25%v of CO, from 12 to 20%v of CO2, from 35 to 55%v of H2, from 15 to 25%v of H2O, from 1 to 4% of N2. It has a temperature from 250 to 500°C. When pure hydrogen is used as reducing gas, the composition of said top reduction gas will be rather composed of 40 to 80%v of H2, 20-50%v of H20 and some possible gas impurities coming from seal system of the shaft or present in the hydrogen stream 40. When the H2 amount in the reducing gas varies and the carbon-bearing liquid 40 is injected, the top gas 20 will have an intermediate composition between the two previously described cases.

In a further embodiment of the method according to the invention, the top reduction gas 20 after a dust and mist removal step in a cleaning device 5, such as a scrubber and a demister, is sent to a separation unit 6 where it is divided into two streams 22,23. This separation unit 6 may be an absorption device, an adsorption device, a cryogenic distillation device or membranes. It could also be a combination of those different devices.

The first stream 22 is a CO2-rich gas which can be captured and used in different chemical processes. In a preferred embodiment, this CO2-rich gas 22 is subjected to a methanation step. The second stream 23 is a H2-rich gas which is sent to a preparation device 7 where it will be mixed with other gas, optionally reformed and heated to produce the reducing gas 11. In a preferred embodiment, the preparation device 7 is a heater.

All the different embodiments previously described may be combined with one another.

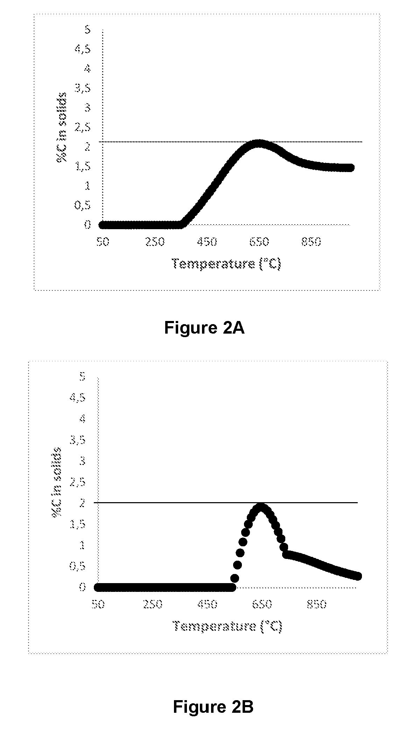

Figure 2A and 2B are curves simulating the evolution of the percentage in weight of carbon into the direct reduced iron product versus temperature when injecting respectively 100kg/ton of DRI of liquid Ethanol (Figure 2A) or 430kg/ ton of DRI of liquid Methanol (Figure 2B). In both cases we can see that when the liquid is injected into the transition zone and/or cooling zone of the furnace, it is possible to reach a carbon content in the solid product of around 2% in weight. The advantage of ethanol is that a smaller quantity is needed compared to methanol and it is more available. The simulation was performed using thermodynamical models.

The method according to the invention allows to obtain a DRI product having required carbon content.

## Claims

1. A method for manufacturing direct reduced iron wherein oxidized iron is reduced in a direct reduction furnace by a reducing gas, said direct reduction furnace comprising a reduction zone, a transition zone and a cooling zone, a carbon-bearing liquid being injected below the reduction zone, and wherein a top reduction gas is captured at the exit of the direct reduction furnace and subjected to at least one separation step so as to be split between a CO2-rich gas and an H2-rich gas, said H2-rich gas being at least partly used as reduction gas.

2. A method according to claim 1 wherein the carbon-bearing liquid is injected at least into the transition zone.

3. A method according to claim 1 wherein the carbon-bearing liquid is injected at least into the cooling zone.

4. A method according to anyone of claims 1 to 3 wherein the carbon-bearing liquid is injected in the transition zone and in the cooling zone.

5. A method according to anyone of the previous claims wherein said carbon-bearing liquid is a biofuel.

6. A method according to anyone of the previous claims wherein said carbon-bearing liquid is liquid alcohol.

7. A method according to anyone of the previous claims wherein said carbon-bearing liquid is ethanol.

8. A method according to anyone of claims 1 to 5 wherein said carbon-bearing liquid is liquid hydrocarbon.

9. A method according to anyone of the previous claims wherein the reducing gas comprises more than 50% in volume of hydrogen.

10. A method according to anyone of the previous claims wherein the reducing gas comprises more than 99% in volume of hydrogen.

11. A method according to claims 9 or 10 wherein the hydrogen of the reducing gas is at least partly produced by electrolysis.

12. A method according to claim 11, wherein said electrolysis is powered by energy that is collected from renewable resources, which are naturally replenished on a human timescale, chosen among, wind, rain, tides, waves, geothermal heat or nuclear sources.

13. A method according to anyone of the previous claims wherein said CO2-rich gas is subjected to an hydrocarbon production step.

## Patentansprüche

1. ) Ein Verfahren zur Herstellung von direktreduziertem Eisen, wobei oxidiertes Eisen in einem Direktreduktionsofen durch ein Reduktionsgas reduziert wird, wobei der Direktreduktionsofen eine Reduktionszone, eine Übergangszone und eine Kühlzone umfasst, eine kohlenstoffhaltige Flüssigkeit unterhalb der Reduktionszone eingespritzt wird, und wobei ein oberes Reduktionsgas am Austritt des Direktreduktionsofens aufgefangen und mindestens einem Trennschritt unterzogen wird, um zwischen einem C02-reichen Gas und einem H2-reichen Gas aufgeteilt zu werden, wobei das H2-reiche Gas zumindest teilweise als Reduktionsgas verwendet wird.

2. ) Ein Verfahren nach Anspruch 1, wobei die kohlenstoffhaltige Flüssigkeit zumindest in die Übergangszone eingespritzt wird.

3. ) Ein Verfahren nach Anspruch 1, wobei die kohlenstoffhaltige Flüssigkeit zumindest in die Kühlzone eingespritzt wird.

4. ) Ein Verfahren nach irgendeinem der Ansprüche 1 bis 3, wobei die kohlenstoffhaltige Flüssigkeit in die Übergangszone und in die Kühlzone eingespritzt wird.

5. ) Ein Verfahren nach irgendeinem der vorhergehenden Ansprüche, wobei die kohlenstoffhaltige Flüssigkeit ein Biokraftstoff ist.

6. ) Ein Verfahren nach irgendeinem der vorhergehenden Ansprüche, wobei die kohlenstoffhaltige Flüssigkeit flüssiger Alkohol ist.

7. ) Ein Verfahren nach irgendeinem der vorhergehenden Ansprüche, wobei die kohlenstoffhaltige Flüssigkeit Ethanol ist.

8. ) Ein Verfahren nach irgendeinem der Ansprüche 1 bis 5, wobei die kohlenstoffhaltige Flüssigkeit flüssiger Kohlenwasserstoff ist.

9. ) Ein Verfahren nach irgendeinem der vorhergehenden Ansprüche, wobei das Reduktionsgas mehr als 50 Volumen-% Wasserstoff umfasst.

10. ) Ein Verfahren nach irgendeinem der vorhergehenden Ansprüche, wobei das Reduktionsgas mehr als 99 Volumen-% Wasserstoff umfasst.

11. ) Ein Verfahren nach Anspruch 9 oder 10, wobei der Wasserstoff des Reduktionsgases zumindest teilweise durch Elektrolyse erzeugt wird.

12. ) Ein Verfahren nach Anspruch 1 1, wobei die Elektrolyse durch Energie betrieben wird, die aus erneuerbaren Ressourcen gesammelt wird, die auf einer menschlichen Zeitrahmen natürlich erneuert werden, ausgewählt aus Wind, Regen, Gezeiten, Wellen, Erdwärme oder nuklearen Quellen.

13. ) Ein Verfahren nach irgendeinem der vorhergehenden Ansprüche, wobei das C02-reiche Gas einem Kohlenwasserstoffproduktionsschritt unterzogen wird.

## Revendications

1. Un procédé de fabrication de fer réduit directement dans lequel du fer oxydé est réduit dans un four de réduction directe par un gaz réducteur, ledit four de réduction directe comprenant une zone de réduction, une zone de transition et une zone de refroidissement, un liquide carboné étant injecté sous la zone de réduction, et dans lequel un gaz de réduction de tête est capté à la sortie du four de réduction directe et soumis à au moins une étape de séparation de manière à être séparé en un gaz riche en C02 et un gaz riche en H2, ledit gaz riche en H2 étant au moins partiellement utilisé comme gaz réducteur.

2. Un procédé selon la revendication 1 dans lequel le liquide carboné est injecté au moins dans la zone de transition.

3. Un procédé selon la revendication 1 dans lequel le liquide carboné est injecté au moins dans la zone de refroidissement.

4. Un procédé selon l'une quelconque des revendications 1 à 3 dans lequel le liquide carboné est injecté dans la zone de transition et dans la zone de refroidissement.

5. Un procédé selon l'une quelconque des revendications précédentes dans lequel ledit liquide carboné est un biocarburant.

6. Un procédé selon l'une quelconque des revendications précédentes dans lequel ledit liquide carboné est un alcool liquide.

7. Un procédé selon l'une quelconque des revendications précédentes dans lequel ledit liquide carboné est de l'éthanol.

8. Un procédé selon l'une quelconque des revendications 1 à 5 dans lequel ledit liquide carboné est un hydrocarbure liquide.

9. Un procédé selon l'une quelconque des revendications précédentes dans lequel le gaz réducteur comprend plus de 50 % en volume d'hydrogène.

10. Un procédé selon l'une quelconque des revendications précédentes dans lequel le gaz réducteur comprend plus de 99 % en volume d'hydrogène.

11. Un procédé selon les revendications 9 ou 10 dans lequel l'hydrogène du gaz réducteur est au moins partiellement produit par électrolyse.

12. Un procédé selon la revendication 1 1, dans lequel ladite électrolyse est alimentée par une énergie qui est collectée à partir de ressources renouvelables, qui sont naturellement reconstituées à l'échelle de temps humaine, choisies parmi le vent, la pluie, les marées, les vagues, la chaleur géothermique ou les sources nucléaires.

13. Un procédé selon l'une quelconque des revendications précédentes dans lequel ledit gaz riche en C02 est soumis à une étape de production d'hydrocarbures.
